# EUROPÄISCHE PATENTANMELDUNG

(11) **EP 1 205 541 A1**
(43) Veröffentlichungstag der Anmeldung: **15.05.2002**
(21) Anmeldenummer: 00124782.4
(22) Anmeldetag: 14.11.2000
(51) Int. Cl.: C12N 5/06

(54) **Verfahren zur Langzeit-Kultivierung organotypischer Slices von Gehirn und anderen Geweben postnataler, insbesondere adulter Säuger**

(71) Anmelder: Giehl, Klaus, Dr., 66111 Saarbrücken (DE)
(72) Erfinder: Giehl, Klaus, Dr., 66111 Saarbrücken (DE)
(74) Vertreter: Patentanwälte Hauck, Graalfs, Wehnert, Döring, Siemons

(57) **Zusammenfassung**

Es wird ein Verfahren zur Langzeit-Kultivierung organotypischer Slices von Gehirn und anderen Geweben postnataler, insbesondere adulter Säuger beschrieben. Bei dem Verfahren wird beispielsweise ein Slice auf eine poröse Membran aufgetragen, Kultivierungsmedium in die Vertiefung einer Aufnahmevorrichtung für die Membran aufgebracht, die mit dem Slice versehene Membran in die Vertiefung eingesetzt und ein gerichteter Sog vom Slice zum Medium erzeugt. Mit diesem Verfahren lassen sich Gewebe-Slices aus allen Körperregionen unabhängig vom Entnahme-Alter langfristig kultivieren.

## Beschreibung

Die vorliegende Erfindung betrifft ein Verfahren zur Langzeit-Kultivierung organotypischer Slices von Gehirn (alle Gehirnareale, z.B. Cortex, Hippocampus, Rückenmark etc.) und anderen Geweben adulter Säuger.

Die Kultivierung postnatalen, insbesondere adulten Gewebes, bereitet große Schwierigkeiten. So war es bis vor kurzem überhaupt nicht möglich, Gehirn-Gewebe von postnatalen Säugern zu kultivieren. Nervengewebs-Kulturen bzw. dem Nervengewebe "äquivalente" Kulturen konnten nur aus embryonalem Nervensystem bzw. von Nervenzell-äquivalenten, Tumoren entstammenden Zell-Linien erfolgen. Die Möglichkeit, adultes Nervengewebe über längere Zeiträume (d.h. länger als 1-2 Tage) in Kultur zu nehmen, wäre aber für die medizinisch/ pharmakologische Forschung von sehr großem Nutzen. So wären z.B. Screens nach wirksamen, Zelltod-verhindernden Substanzen im Rahmen der Erforschung neurodegenerativer Erkrankungen (Morbus Alzheimer, Morbus Parkinson, Amyotrophe Lateralsklerose etc.) bevorzugt an den Geweben durchzuführen, auf die letzlich ein Medikament wirken soll, nämlich auf bestimmte adulte Gehirn-Areale, und nicht wie bisher an Zell-Linien oder Primärkulturen embryonalen Nervengewebes.

Seit den frühen 80iger Jahren ist es bekannt, organotypische Slice(Scheiben)-Kulturen von postnatalem Gehirn anzulegen. Bei dieser Technik wird die zyklische Exposition der Slices mit Luft und Kulivierungsmedium in rotierenden, sogenannten "Roller-Kulturen" ausgenutzt. Die Slices werden auf einer im "Roller-Tube" fixierten Glasunterlage in eine dreidimensionale Matrix (Plasma Clot) eingebettet, wodurch die Slices während der Rotation in Position gehalten werden. Mit diesen Kulturtechniken gelingt es, Gehirn-Slices von max. 14 Tage alten Tieren (entsprechend 14 postnatalen Tagen = P14) über mehrere Tage bis Wochen in Kultur zu halten. Dieses Verfahren ist beispielsweise in Gähwiler BH (1988) "Organotypic cultures of neural tissue", Trends in Neurosience 11, 484-490 beschrieben.

Auf der Basis dieses bekannten Verfahrens wurde von Stoppini eine einfache Methode der Slice-Kultur auf CM-Membranen entwickelt (Stoppini et al. (1991) "A simple method for organotypic cultures of nervous tissue", Journal of Neuroscience Methods 37, 173-182). Bei dieser Methode werden Slices auf einer Membran (Millicell-CM, Millipore) gelagert, die in einen Plastikring eingespannt ist (CM-Insert). Das Medium befindet sich außerhalb/unterhalb des CM-Inserts, die Exposition des Slices mit Medium erfolgt hierbei von unten durch die CM-Membran. Der pH-Wert darf 7,2 nicht überschreiten, und die Slices dürfen nicht mit Kultivierungsmediumtropfen bedeckt sein. Überschüssiges, über dem Slice befindliches Kultivierungsmedium muß von oben abgesaugt werden. Kultivierungsmedium und CM-Membran befinden sich auf einem Niveau, was bei den gewählten Kulturbedingungen durch die Verwendung von 1 ml Kultivierungsmedium erreicht ist. Nach wenigen Tagen adhärieren die Slices an die CM-Membranen.

Mit der Methode nach Stoppini können Slices von Tieren (Ratten, Mäuse) maximal des Stadiums von ca. P30 kultiviert werden, d.h. die Tiere sind zwar postnatal, aber noch nicht adult, da sie noch nicht geschlechtsreif sind (Geschlechtsreife bei Ratten: ab P58). Mit dieser Technik läßt sich somit kein adultes Nervengewebe kultivieren.

Der vorliegenden Erfindung liegt die Aufgabe zugrunde, ein Verfahren zur Langzeit-Kultivierung organotypischer Slices von Gehirn und anderen Geweben adulter Säuger anzugeben.

Ferner soll ein Verfahren angegeben werden, das die Erfolgsquote der Kulturen im Vergleich zu dem vorstehend beschriebenen Verfahren nach Stoppini aus jungem postnatalen Gehirn und anderen Geweben verbessert.

Diese Aufgabe wird erfindungsgemäß durch ein Verfahren gemäß Patentanspruch 1 oder gemäß Patentanspruch 2 gelöst.

Bei einer weiteren Alternative der Erfindung wird eine poröse Membran ohne Slice auf das Medium aufgebracht, der gerichtete Sog von der Membran zum Medium erzeugt und der Slice auf die poröse Membran aufgetragen. Bei noch einer Alternative wird die poröse Membran ohne Slice auf einer planen Fläche oder in der Vertiefung einer Aufnahmevorrichtung für die Membran angeordnet, das Kultivierungsmedium unter der Membran angeordnet, der gerichtete Sog von der Membran zum Medium erzeugt und der Slice auf die poröse Membran aufgetragen. Auch hier wird schließlich ein gerichteter Sog vom Slice zum Medium erzielt.

Bei seinen Untersuchungen zur Konzipierung der vorliegenden Erfindung, bei denen Slice-Kultur-Experimente an postnatalem Gehirn nach der Methode von Stoppini durchgeführt wurden, hat der Erfinder folgendes festgestellt:

Es besteht eine negative Korrelation zwischen der Größe von Medium-Tropfen über dem Slice und der Vitalität der Slices, d.h. je größer der Tropfen, desto degenerierter/avitaler werden die Slices. Die Tendenz der Slices, an die CM-Membran zu adhärieren, nimmt von P15 bis 25 zusehends ab und ist bei Slices von älteren Stadien quasi nicht mehr gegeben. Eine deutliche Erhöhung des Parameters "Slice-Degeneration" und "Tropfen-Bildung über dem Slice" erfolgte bei Slices von Gehirnen des Stadiums P15 bis P25. Kulturen von Gehirn-Slices der Stadien über P25 waren nach dem Stoppini-Protokoll nicht mehr möglich. Diese Kulturen zeigten primär Degeneration und Slice-Fragmente, die in/auf Medium-Tropfen schwammen. Während einerseits Medium-Tropfen über dem Slice dessen Degeneration erhöhen, wird andererseits auch durch die Degeneration die Tendenz zur Tröpfchenbildung erhöht. Dies könnte dadurch erklärt werden, daß durch lytische Prozesse im Bereich des Slices die Osmolarität lokal erhöht wird, was zu einem Einstrom von Medium in diesen Bereich führt. Auch wenn streng darauf geachtet wird, Tropfen-Bildung über dem Slice zu vermeiden/reduzieren, degenerieren Slices von Stadien älter als P25. Was hierbei auffällt ist eine gänzlich fehlende Adhärenz und daraus resultierende Fixierung der Slices an die CM-Membran. Dies wird beispielsweise bei Erschütterung der Kulturschalen deutlich: die Slices bewegen sich dann auf den CM-Membranen.

Aus diesen Beobachtungen gelangte der Erfinder zu der Erkenntnis, daß eine abnehmende Adhäsivität der Slices und eine hieraus resultierende fehlende Fixierung der Slices an der Unterlage (CM-Membran) sowie eine zunehmende Degeneration/Tröpfchenbildung bei zunehmend älteren Stadien der Slices wesentliche Ursachen für das Scheitern von Kulturen älterer Stadien sind.

Mit der erfindungsgemäßen Lösung werden diese Ursachen behoben. Durch einen vom Präparat durch die Membran ins Medium gerichteten leichten Sog (d.h. vom Präparat zum Medium hin gerichteten Druckgradienten) wird diesen Ursachen entgegengewirkt. Dieser Sog (Druckgradient) "saugt" innerhalb weniger Minuten Flüssigkeitstropfen ab, die auf die Membran oder auf den Gewebe-Slice aufgetragen werden. Die Gewebe-Slices werden durch den Sog an die Membran angesaugt und sind dadurch in ihrer Position fixiert. Es besteht keine Alters/Stadienbegrenzung bezüglich der Gewebe-Slices, die in Kultur genommen werden können. D.h. mit diesem Verfahren können Gewebe-Slices von frühen postnatalen Stadien bis hin ins Senium in Kultur genommen werden.

Die mit der erfindungsgemäßen Kultur-Technik kultivierten Slices sind für mindestens zwei Wochen vital. Dieser Zeitraum würde für die gängigen, in der Pharma-Industrie relevanten funktionellen Assays, wie beispielsweise Überlebens/Zelltod-Assays, ausreichen.

Slices von adulten Tieren (d.h. über P58) adhärieren im Normalfall nicht an die Membranen, auch nicht bei langen Kultur-Zeiten, sind aber bei der erfindungsgemäßen Kultivierung eben durch den Sog an ihre Unterlage fixiert. Dies zeigt sich dadurch, daß sich der Slice als Ganzes unmittelbar nach Aufhebung des Soges mobilisiert. Die für das Überleben der Slices offensichtlich notwendige Fixierung an eine Unterlage wird also lediglich durch den leichten Sog erreicht.

Der entscheidende Parameter für die Kultivierung adulten Gewebes ist somit der vom Slice ins Medium gerichtete Sog. Hierdurch ist es erstmals möglich geworden, organotypische Slice-Kulturen von adultem Gehirn und anderen adulten Geweben über einen Zeitraum von mehr als 1-2 Tagen durchzuführen.

Bei dem erfindungsgemäßen Verfahren wird vorzugsweise als poröse Membran eine Membran mit einer Porengröße im Mikrometerbereich verwendet. Speziell findet als poröse Membran eine handelsübliche CM-Membran eines CM-Inserts Anwendung.

Erfindungsgemäß bestehen mehrere Möglichkeiten, einen gerichteten Sog in den Slice-Kulturen zu erzeugen. Bei einer ersten Variante des erfindungsgemäßen Verfahrens wird Kultivierungsmedium mit einem Volumen in eine Vertiefung oder auf eine plane Fläche (z.B. Objektträger) ein-/aufgebracht, das geringer ist als das Volumen (V) der Vertiefung vom Boden bis zur Position der porösen Membran bzw. das Volumen (V), das durch die Auflagefläche (F) des Mediums auf der planen Fläche bis zur Position der porösen Membran definiert ist. Vorzugsweise wird dabei das Kultivierungsmedium mit einem Volumen eingebracht, das 50-75 % des Volumens (V) beträgt.

Bei diesem Verfahren wird die Oberflächenspannung des Kultivierungsmediums ausgenutzt. Vorzugsweise werden die Gewebe-Slices hierbei auf einer CM-Membran kultiviert, die in einen Plastikring eingespannt ist (CM-Insert). Das CM-Insert mit Slice befindet sich zur Kultur in der Vertiefung der Aufnahmevorrichtung für die Membran, insbesondere einem zylinderförmigen Well (kreisförmige Grundfläche, bestimmte Höhe h). Alternativ kann das Insert auch über einen Medium-tropfen auf einer planen Fläche gestellt werden oder in beiden Fällen unter das abgestellte Insert eingebracht werden. Das CM-Insert berührt zweckmäßigerweise den Boden des Wells nicht direkt, sondern steht auf kleinen Füßchen. Durch den hierdurch bestimmten Abstand H der CM-Membran von der Grundfläche des Wells wird ein Volumen V = π(D/2)²H (für Variante in Vertiefung) bzw. V = F·H (für Variante auf planer Fläche) definiert, wobei D/2 der Radius der Well-Grundfläche und F die Fläche ist, die der Mediumtropfen auf der planen Fläche einnimmt. Zur Erzeugung eines Soges auf den Slice muß das Volumen des in ein Well eingegebenen Mediums immer kleiner sein als das Volumen V. Hierdurch bildet sich eine in bezug auf den Slice nach unten gerichteter hydrostatischer Druck aus, der eine "Tropfenbildung" über dem Slice vermeidet und die Slices an die CM-Membran ansaugt.

In der Praxis wird Kultivierungsmedium mit dem gewünschten optimalen Volumen tropfenförmig in die Mitte des Wells auf die plane Fläche aufgetragen. Danach wird das CM-Insert auf den Mediumtropfen gestellt. Da das Kultivierungsmediumvolumen kleiner ist als V, bildet sich ein nach unten zeigender Medien-Konus am Rande des CM-Inserts aus, während die "Spitze" des Medientropfens vollständig in Kontakt mit der Unterfläche der CM-Membran liegt. Für einen Wechsel des Kultivierungsmediums wird dieses Procedere an neuen Well-Schalen/planen Flächen wiederholt, d.h. es werden lediglich die Slice-enthaltenden Inserts von der alten in die neue Kulturvorrichtung transferiert.

Ist das Kultivierungsmediumvolumen größer als V, bilden sich in jedem Fall Medientropfen über den Slices. Dies führt um so mehr zur Degeneration der Slices, je später postnatal sie sind. Bei adulten Slices bilden sich Tropfen im Bereich der Slices, und die Slices degenieren. Da adulte Slices nicht adhärent sind, schwimmen sie in/auf diesen Tropfen. Dieses und der/die Grad/Geschwindigkeit der Degeneration sind um so größer/schneller, je größer das Kultivierungsmediumvolumen im Verhältnis zu V ist.

Bei einer weiteren Variante des erfindungsgemäßen Verfahrens wird der gerichtete Sog mittels Unterdruckbeaufschlagung über eine Pumpe erzeugt. Hierbei wird vorzugsweise der Boden der Vertiefung oder die plane Fläche als flexible Membran ausgebildet, wobei beispielsweise der als flexible Membran ausgebildete Boden oder die Fläche zur Unterdruckerzeugung entweder mechanisch oder durch von einer Vakuumpumpe erzeugten Unterdruck bewegt wird. Die flexible, den Boden oder die Fläche bildende Membran kann somit entweder durch mechanische Verankerung von der den Slice tragenden Membran wegbewegt oder direkt an einen vorzugsweise steuerbaren, durch eine Vakuumpumpe erzeugten Unterdruck angeschlossen werden. Durch beide Möglichkeiten lassen sich über die Steuerung der Größe des Abstandes "Scheitel der flexiblen Membran - Niveau der den Slice tragenden Membran" bei konstantem Medienvolumen die Größen des auf den Slice einwirkenden Soges verändern. Letztendlich wird aber auch bei dieser Verfahrensvariante die Oberflächenspannung des Kultivierungsmediums ausgenutzt.

Bei dem erfindungsgemäßen Verfahren werden vorzugsweise Slices mit einer Dicke von 100-400 µm verwendet. Auch größere Slice-Dicken, wie z.B. die von Stoppini üblicherweise verwendeten 400-500 µm, können verwendet werden.

Was die übrigen Kulturbedingungen anbetrifft, so können bei Durchführung des erfindungsgemäßen Verfahrens übliche Bedingungen Anwendung finden, beispielsweise pH-Wert 7,0-7,2, Temperatur über 30 °C etc.

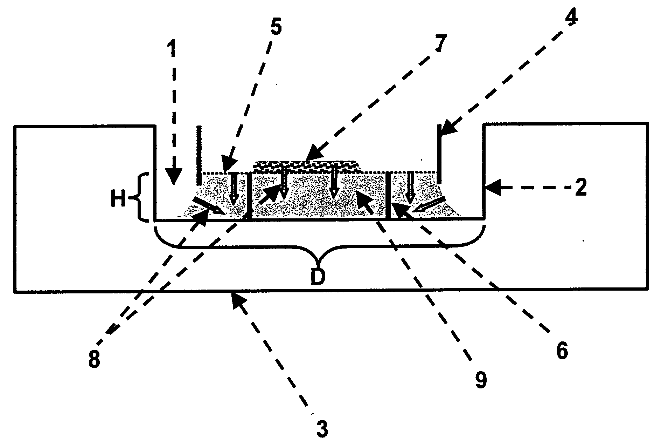

Das erfindungsgemäße Verfahren wird nachfolgend anhand eines Ausführungsbeispieles in Verbindung mit der Zeichnung im einzelnen erläutert. Die einzige Figur zeigt auf schematische Weise einen in einen Well eingesetzten CM-Insert mit einem auf der CM-Membran befindlichen Slice, wobei sich unterhalb der Membran ein Konus des Kultivierungsmediums ausgebildet hat.

Der in der Figur im Querschnitt gezeigte Well 1 befindet sich in einer Multischale 3 mit insgesamt sechs Vertiefungen. Der Well 1 besitzt eine zylindrische Begrenzungswand 2. Derartige Multischalen sind im Handel erhältlich.

In den Well 1 ist ein handelsüblicher CM-Insert 4 mit CM-Membran 5 eingebracht. Die Grundfläche des Wells 1 und des CM-Inserts ist kreisförmig. Der Durchmesser (D) der Well-Grundfläche und der Abstand H von der Well-Grundfläche bis zur CM-Membran sind angedeutet. Auf der CM-Membran 5 befindet sich ein Slice 7. Das CM-Insert ruht mit Füßen 6 auf der Grundfläche des Wells. Unterhalb der CM-Membran 5 befindet sich ein Kultivierungsmedium 9, das konusförmig ausgebildet ist (der Konus erweitert sich vom Insert weg nach unten). Der vom Slice nach unten ausgeübte Sog ist durch Pfeile 8 gekennzeichnet.

Es wurde folgendes Ausführungsbeispiel durchgeführt. Gehirn-Slices wurden separiert, je nach Bedarf präpariert und mit einem Spatel auf eine handelsübliche CM-Membran aufgetragen (CM-Insert: Culture Plate Inserts, MILLICELL TM, 0,4 µm Porengröße, 30 mm Durchmesser, SIGMA Product Nr. Z 35, 499-6). 0,75 ml Kultivierungsmedium wurden mittig in den in der Figur dargestellten Well einer Multischale (NUNC Product Nr. 152795, 133 x 88 mm) bei 36 °C und 5 % CO₂ eingebracht. Die eingebrachte Menge entsprach unter Berücksichtigung des Well-Volumens bis zur Membranposition (D/2)²H der für die Ausbildung des in der Figur dargestellten Konus erforderlichen Menge. Danach wurde das CM-Insert mit seinen Füßen 6 auf den Medium-Tropfen gestellt. Es bildete sich dabei der in der Figur dargestellte Konus aus, wobei die Spitze des Medientropfens vollständig in Kontakt mit der Unterfläche der CM-Membran lag.

Auf die vorstehend geschilderte Weise wurde eine Vielzahl von Slices kultiviert.

Die Vitalität der in Kultur genommenen Slices wurde z.B. mit handelsüblichen Assays (LIVE/DEAD Assay für Säuger-Zellen, Molecular Probes) und herkömmlichen Cresylviolett- und Hämatoxilin-Eosin (HE)-Färbungen der gesamtesn Slices und von 10 µm Kryoschnitten der Slices bestätigt und analysiert. Zusätzlich erfolgte an Gehirn-Slices eine auf neuronalen Tracern basierende Quantifizierung des Überlebens bestimmter Subpopulationen von Neuronen, die in außerhalb des Slices liegende Gehirn-Areale projizieren. Die Analysetechniken bestätigten, daß die kultivierten Slices für mindestens zwei Wochen überlebten. Man kann hierbei zwischen Initialphase (erste 2 Tage in Kultur) und Postinitialphase (sich der Initialphase anschließende Phaase) unterscheiden. In der Initialphase wird bei bisher allen untersuchten Zelltypen ein Absterben von 5-30 % der Zellen beobachtet. In der Postinitialphase kommt es je nach Zelltyp zu einer Verlangsamung (Stabilisierung) oder Progredienz der Zelltodkinetik.

Beispielsweise starben im Neocortex in dieser Postinitialphase corticale Schichten 2/3 Neurone (hauptsächlich Interneurone) deutlich langsamer ab als corticospinale Neurone (corticale Schicht 5 Motoneurone, die ins Rückenmark - also außerhalb des Slices - projizieren). Dies verdeutlichte hauptsächlich zweierlei Aspekte:
a) Der in den Slices beobachtet Zelltod reflektierte nicht ausschließlich eine allgemeine "Degenerations-Tendenz" der Slices über die Zeit, sondern ein Zelltyp/Funktionsspezifisches Zelltod-Geschehen in einer organotypischen Situation.
b) Das Vorhandensein einer sich über mehrere Tage erstreckenden Zelltod-Kinetik in den entsprechenden, unter Sog gehaltenen Kulturen bietet die Basis für eine pharmakologisch relevante Screen-Möglichkeit von Substanzen, die Zelltod verlangsamen/verhindern (degenerative Erkrankungen) oder Zelltod beschleunigen (Malignome, proliferative Erkrankungen) in einer organotypischen Situation.

An Cresylviolett- und HE-gefärbten Slices aller Stadien nach "0 Tagen in Kultur" (0 DIV [von "day in vitro"]), 1 DIV, 5 DIV, 7 DIV und 14 DIV wurde gezeigt, daß in Slices aller untersuchten Gewebe die Gewebearchitektur und gewebespezifischen Zelltypen nach lichtmikroskopischen Kriterien erhalten blieben. Es kam lediglich zu einer geringfügigen Abflachung der Slices.

Mit dem dargestellten Verfahren lassen sich Gewebe-Slices aus allen Körperregionen und Organen (z.b. Leber, Niere, Lunge) unabhängig vom Entnahme-Alter langfristig kultivieren. Es sind also sämtliche postnatale Stadien bis hin zum Senium abgedeckt.

## Patentansprüche

1. Verfahren zur Langzeit-Kultivierung organotypischer Slices von Gehirn und anderen Geweben postnataler, insbesondere adulter Säuger mit den folgenden Schritten:
(a) Auftragen eines Slices auf eine poröse Membran;
(b) Aufbringen von Kultivierungsmedium auf eine plane Fläche oder in die Vertiefung einer Aufnahmevorrichtung für die Membran;
(c) Aufbringen der mit dem Slice versehenen Membran auf das Medium;
(d) Erzeugen eines gerichteten Soges vom Slice zum Medium.

2. Verfahren zur Langzeit-Kultivierung organotypischer Slices von Gehirn und anderen Geweben postnataler, insbesondere adulter Säuger mit den folgenden Schritten:
(a) Auftragen eines Slices auf eine poröse Membran;
(b) Anordnen der mit dem Slice versehenen Membran auf einer planaren Fläche oder in der Vertiefung einer Aufnahmevorrichtung für die Membran;
(c) Anordnen eines Kultivierungsmediums unter der Membran;
(d) Erzeugen eines gerichteten Soges vom Slice zum Medium.

3. Verfahren nach Anspruch 1 oder 2, **dadurch gekennzeichnet, daß** als poröse Membran eine Membran mit einer Porengröße im Mikrometerbereich verwendet wird.

4. Verfahren nach einem der vorangehenden Ansprüche, **dadurch gekennzeichnet, daß** als poröse Membran eine CM-Membran eines CM-Inserts verwendet wird.

5. Verfahren nach einem der vorangehenden Ansprüche, **dadurch gekennzeichnet, daß** Kultivierungsmedium mit einem Volumen auf eine plane Fläche (z.B. Objektträger) aufgebracht wird, das geringer ist als das Volumen (V), das durch die Auflagefläche (F) des Mediums auf der planen Fläche und die Höhe (H) von der planen Fläche bis zur Position der porösen Membran bestimmt ist.

6. Verfahren nach einem der Ansprüche 1-4, **dadurch gekennzeichnet, daß** die Membran im Abstand vom Boden der Vertiefung angeordnet wird.

7. Verfahren nach einem der vorangehenden Ansprüche, **dadurch gekennzeichnet, daß** Kultivierungsmedium mit einem Volumen in die Vertiefung eingebracht wird, das geringer ist als das Volumen (V) der Vertiefung vom Boden bis zur Position der porösen Membran.

8. Verfahren nach Anspruch 7, **dadurch gekennzeichnet, daß** ein optimiertes Kultivierungsmediumvolumen 50-75 % des Volumens (V) beträgt.

9. Verfahren nach einem der vorangehenden Ansprüche, **dadurch gekennzeichnet, daß** das Medium tropfenförmig in die Mitte der Vertiefung bzw. auf die plane Fläche aufgetragen wird.

10. Verfahren nach einem der vorangehenden Ansprüche, **dadurch gekennzeichnet, daß** der gerichtete Sog mittels Unterdruckbeaufschlagung über eine Pumpe erzeugt wird.

11. Verfahren nach einem der vorangehenden Ansprüche, **dadurch gekennzeichnet, daß** der Boden der Vertiefung oder die plane Fläche als flexible Membran ausgebildet wird.

12. Verfahren nach Anspruch 11, **dadurch gekennzeichnet, daß** der als flexible Membran ausgebildete Boden oder die Fläche zur Unterdruckerzeugung mechanisch oder durch von einer Vakuumpumpe erzeugten Unterdruck bewegt wird.

13. Verfahren nach einem der vorangehenden Ansprüche, **dadurch gekennzeichnet, daß** Slices mit einer Dicke von 100-400 µm verwendet werden.
